**Europäisches Patentamt**

**European Patent Office**

**Office européen des brevets**

(11) Veröffentlichungsnummer: **0 273 127**
**A2**

# EUROPÄISCHE PATENTANMELDUNG

(21) Anmeldenummer: **87115585.9**

(51) Int. Cl.⁴ **C12N 15/00**

(22) Anmeldetag: **23.10.87**

Die Mikroorganismen sind bei der Deutsche Sammlung von Mikroorganismen und bei den American Type Culture Collection unter den Nummern 3611P, 1607, 3858P, 3859P, 3860P, 3861P, 3862P, CRL 1658, CRL 9078 hinterlegt worden.

(30) Priorität: **24.10.86 DE 3636287**

(43) Veröffentlichungstag der Anmeldung:
**06.07.88 Patentblatt 88/27**

(84) Benannte Vertragsstaaten:
**AT BE CH DE ES FR GB GR IT LI LU NL SE**

(71) Anmelder: **Boehringer Mannheim GmbH**
**Sandhofer Strasse 112-132**
**D-6800 Mannheim-Waldhof(DE)**

(72) Erfinder: **Hirth, Peter, Dr.**
**Winkstrasse 6**
**D-8000 München 70(DE)**
Erfinder: **Buckel, Peter, Dr.**
**Eichenstrasse 19**
**D-8139 Bernried(DE)**

(74) Vertreter: **Huber, Bernhard, Dipl.-Chem. et al**
**Patentanwälte H. Weickmann, Dr. K. Fincke**
**F.A. Weickmann, B. Huber Dr. H. Liska, Dr. J.**
**Prechtel Möhlstrasse 22 Postfach 860 820**
**D-8000 München 86(DE)**

(54) **Verfahren zur gentechnologischen Herstellung von Stoffwechselprodukten in Eukaryontenzellen.**

(57) Zur gentechnologischen Herstellung von Stoffwechselprodukten in Eukaryontenzellen durch Einführung eines das gewünschte Stoffwechselprodukt kodierenden Fremdgens mit Hilfe eines retroviralen Provirus als Vektor in eine Eukaryontenzelle und Expression des durch das Fremdgen kodierten Produktes, baut man das zu exprimierende Fremdgen in einen in einem Vektor integrierten Provirus ein, der keine funktionsfähigen Gene für die Replikations-und Verpackungsfunktionen gag, pol oder/und env mehr enthält, führt den so erhaltenen, den Provirus enthaltenden Vektor und einen weiteren, mindestens eine Markerfunktion, aber keine mit den Verpackungsfunktionen interferierenden Gene enthaltenden Vektor in eine eukaryontische Zelle ein, die im Genom die retroviralen Gene der Replikations-und Verpackungsfunktionen gag, pol oder/und env enthält, wobei Provirus und retrovirale Gene im Genom zusammen einen kompletten Retrovirus kodieren müssen, kloniert die transfektierte Zelle und selektioniert nach Markerfunktion und gewünschtem Stoffwechselprodukt, infiziert mit den im Wachstumsmedium der selektionierten Zellen gebildeten Retroviren eine Eukaryontenzelle und gewinnt das gewünschte Stoffwechselprodukt aus der infizierten Zelle oder ihrem Züchtungsmedium.

FIG.1

Xerox Copy Centre

## Verfahren zur gentechnologischen Herstellung von Stoffwechselprodukten in Eukaryontenzellen

Die Erfindung betrifft ein Verfahren zur gentechnologischen Herstellung von Stoffwechselprodukten in Eukaryonten-Zellen.

Es ist bekannt die gentechnologische Herstellung von Stoffwechselprodukten in Eukaryonten-Zellen durch Einführung eines das gewünschte Stoffwechselprodukt codierenden Fremdgens mit Hilfe eines Vektors, der in die Eukaryonten-Zelle eingebracht wird, durchzuführen, wobei anschließend das durch das Fremdgen codierte Produkt von der Zelle exprimiert wird. In der Praxis hat sich jedoch gezeigt, daß diese Verfahren in der Regel zu unbefriedigenden Ergebnissen führen, sowohl hinsichtlich der Expressionsrate als auch hinsichtlich der Weiterzüchtbarkeit der durch den Vektor infizierten Eukaryonten-Zelle ohne genetische Veränderungen.

Man hat daher bereits versucht, derartige Verfahren zu verbessern, in dem man als infizierenden Vektor Retroviren verwendet, welche zu einer Integration ins Eukaryonten-Genom befähigt sind.

Retroviren sind Viren mit einem einzelsträngigen RNA-Genom, die für ihre Replikation Proviren in Form eines DNA-Intermediats benutzen und in Vögeln und Säugetieren vorkommen. Hierbei dringt die virale RNA in die Zelle ein und wird mittels reverse Transkriptase in doppelsträngige lineare DNA umgeschrieben. Diese lineare DNA wird zu einem Provirus zirkulär geschlossen und anschließend an bestimmte Stellen im Zell-Genom integriert.

Beispiele für derartige Retroviren sind SNV, MMTV, MoMLV, MSV und SSFV.

Das virale Genom hat im allgemeinen eine Länge von ca. 10 Kilobasen (kB) und enthält, wenn es als Provirus in DNA integriert vorliegt, an beiden Enden Retrovirus-spezifische Sequenzen (LTR = Long Terminal Repeat) und dazwischen Gene für Strukturproteine (gag und env), ein Gen für die reverse Transkriptase (pol) und manchmal ein Onkogen.

Nach Integration des viralen Genoms als Provirus in das Zellgenom werden die oben genannten Gene transkribiert, wobei neue virale RNA entsteht, welche durch die ebenfalls exprimierten Strukturproteine umhüllt und ggf. als kompletter Retrovirus aus der Zelle ausgeschleust wird.

Die Verwendung von Retroviren bei der gentechnologischen Herstellung von heterologen Proteinen, wie z.B. Interferon, t-PA (Gewebs-Plasminogenaktivator) oder Faktor VII, könnte zu einer wesentlichen Verbesserung dieser Verfahren führen. Mit Hilfe der Retroviren könnte nämlich das Gen des heterologen Proteins in das Genom der Zelle integriert werden, so daß der Stoffwechsel der infizierten Zelle weniger gestört wird als bei Verwendung von extrachromosomalen Vektoren für die Einführung des gewünschten codierenden Gens.

Es wurde bereits versucht, Retroviren zur Integration von heterologen Genen, insbesondere Proteine codierenden Genen, in Eukaryonten-Genom zu verwenden.

So wird in Science (1982), 216, 812-820 vorgeschlagen, das zu exprimierende Gen in das Genom eines retroviralen Provirus zu integrieren (vgl. auch Genetic Engineering, 7, 235 bis 262).

Hierzu wird auf DNA-Ebene, unter Beibehaltung der long terminal repeats (LTR's), die zentrale Region des Virus, welche gag, pol und env enthält, teilweise herausgeschnitten und durch die Fremd-DNA ersetzt. Als Fremdgene wurden z.B. in USP 4,405,712 V-mos, in Cell 37 (1984), 1053-1062 neo und in Molec. Cell. Biol. (1983), 3, 1123-1132 gpt beschrieben. Diese modifizierten Retroviren können, da ihnen die Replifikationsfunktionen (pol und env) fehlen, allerdings ohne weiteres nicht mehr verpackt bzw. vermehrt werden. Hierzu benötigen diese defekten Viren Helfer-Retroviren. Diese werden in Eukaryonten-Zellen integriert und stellen dort die für die Verpackung notwendigen Funktionen zur Verfügung (z.B. Cell. 33 (1983), 153-159).

Diese Verfahren sind jedoch nur dann geeignet, wenn auf einfache Weise und einzeln diejenigen Zellen bzw. Klone selektiert werden können, in die das Fremdgen integriert wurde. Für die Gene von Humanproteinen, wie Interferon oder t-PA ist jedoch ein solches empfindliches Selektionsverfahren nicht bekannt.

Der Erfindung liegt daher die Aufgabe zugrunde ein Verfahren zur gentechnologischen Herstellung von Stoffwechselprodukten in Eukaryonten-Zellen zu schaffen, welches eine effektive Integration des das gewünschte Produkt codierenden Fremdgens über retrovirale Funktionen, sowie eine Selektion der dabei erhaltenen transfektierten Zellen ermöglicht.

Gelöst wird diese Aufgabe gemäß der Erfindung durch ein Verfahren zur gentechnologischen Herstellung von Stoffwechselprodukten in Eukaryonten-Zellen durch Einführung eines das gewünschte Stoffwechselprodukt codierenden Fremdgens mit Hilfe eines retroviralen Provirus als Vektor in eine Eukaryonten-Zelle und Expression des durch das Fremdgen codierten Produktes, welches dadurch gekennzeichnet ist, daß man

a) das zu exprimierende Fremdgen in einen in einem Vektor integrierten Provirus einbaut, der keine funktionsfähigen Gene für die Replikations- und Verpackungsfunktionen gag, pol oder/und env mehr enthält,

b) den so erhaltenen, den Provirus enthaltenden Vektor und einen weiteren, mindestens eine Markerfunktion, aber keine mit den Verpackungsfunktionen interferierende Gene enthaltenden Vektor in eine Zelle einführt, die im Genom die retroviralen Gene der Replikations-und Verpackungsfunktionen gag, pol oder/und env enthält, wobei Provirus und retrovirale Gene im Genom zusammen einen kompletten Retrovirus kodieren müssen,

c) die transfektierte Zelle kloniert und nach Markerfunktion und gewünschtem Stoffwechselprodukt selektioniert,

d) mit den im Wachstumsmedium der selektionierten Zellen gebildeten Retroviren eine Eukaryonten-Zelle infiziert und das gewünschte Stoffwechselprodukt aus der infizierten Zelle oder ihrem Züchtungsmedium gewinnt.

Der in Stufe a des erfindungsgemäßen Verfahrens verwendete Provirus besteht vorzugsweise nur noch aus den Anfangs-und Endsequenzen, also dem 5'-Terminus des Provirus mit den Sequenzen des LTR (Long Terminal Repeat) und den Sequenzen des 3'LTR, wobei diese Anfangs-und Endsequenzen in der gleichen Orientierung wie im kompletten Provirus vorliegen. Das zu exprimierende Gen ist zwischen diesen beiden Endsegmenten eingebaut. Damit fehlen dem verwendeten Provirus die Funktionen gag, pol und env vollständig. Es ist jedoch auch möglich, daß eine dieser Funktionen noch vorhanden ist und dafür in der Zelle fehlt, die in Stufe b des erfindungsgemäßen Verfahrens mit dem Provirus und dem Markervektor transfektiert wird. Mit anderen Worten sollen in der transfektierten Zelle die Replikations-und Verpackungsfunktionen gag, pol und env jeweils nur ein einziges Mal vorliegt, entweder im neu eingeführten Provirus oder im Genom der eingesetzten Zelle.

Beispiele für im erfindungsgemäßen Verfahren verwendbare Retroviren sind MoMLV (Murine leukemia virus), MMTV (mouse mammary tumor virus), SNV (spleen necrosis virus), SSFV (spleen focus forming virus) oder MSV (Mouse sarcom virus). Zweckmäßigerweise werden für die Erfindung bereits bekannte Vektoren verwendet in welche die retroviralen Elemente als Provirus eingebaut sind. Beispiele hierfür sind pZIP neo SV (DSM 3859 P) (Mulligan et al., Cell, Vol. 37, 1053-1062) und MVGT (ATCC 37191), sowie die in Mol. and Cell biol. 4 (1984), 2289-2297 und Cell 39 - (1984) 459-467 beschriebenen Vektoren. Als Basisvektor, in welchen die Anfangs-und Endglieder des Provirus eingebaut sind, kommen beispielsweise die bekannten, in der Gentechnologie verwendeten Plasmide in Betracht, wie pBR 322. Besonders bevorzugt wird der Vektor pZIP neo SV, in welchem bereits der 5'-Terminus des MoMLV-Provirus als Hind III-Bam HI Fragment und das 3'-Terminus-Element als Xho I-Eco R I Fragment in einem pBR 322 Plasmid eingebaut vorliegt.

Zur Verwendung im Verfahren der Erfindung wird aus dem Vektor pZIP neo SV der zwischen den beiden retroviralen Endsequenzen liegende Teil herausgeschnitten, wozu man zweckmäßig den Vektor sowohl mit Bam HI als auch mit Xho I spaltet. Durch Behandlung der Restriktionsstellen mit Klenow Polymerase läßt sich ein DNA-Fragment isolieren, das nach Ligation und Transformation in E.coli HB 101 (DSM 1607) einen Vektor ergibt, bei dem das 5'-und das 3'-Element des Provirus direkt miteinander verknüpft vorliegen. Hierbei entsteht an der Verknüpfungsstelle dieser beiden Endsequenzen eine Xho I Stelle, die sonst im Vektor nicht vorhanden ist. Diese Stelle läßt sich mit Xho I leicht spalten und eignet sich daher zur Insertion der zu exprimierenden Fremdgene.

In analoger Weise lassen sich auch andere Vektoren als Träger für den Provirus und das in letzteren eingeführte Fremdgen verwenden. Anhand der bekannten Schnittstellen der Restriktionsendonukleasen können beliebige Vektoren je nach der gewünschten Zielzelle ausgesucht und die Verfahrensstrategie zur Einführung von Provirus und Fremdgen festgelegt werden. Diese Methoden sind dem Fachmann an sich bekannt und bedürfen hier keiner näheren Erläuterung.

Der in Stufe a des erfindungsgemäßen Verfahrens verwendete, in einen Vektor integrierte Provirus, der die Gene gag, pol oder/und env nicht mehr enthält, wird zweckmäßigerweise erst in einem geeigneten Prokaryonten, bei Verwendung von Plasmid pBR 322 als Träger beispielsweise in E.coli, bei Verwendung eines anderen Vektors jeweils in dem hierzu geeigneten Prokaryonten vervielfältigt, um für Stufe b ausreichende Mengen an Provirus enthaltenden Vektor zu erzeugen.

Als Markervektor wird zweckmäßig ebenfalls ein Plasmid verwendet, welches für die Zielzelle geeignet ist und eine der bekannten Markerfunktionen enthält, also beispielsweise ein oder mehrere Antibiotikaresistenzgene, Oncogen, Hygromycin (Molec and Cell Biology 1984, Vol. 4 No. 12, p 2929-2931, Gene 25 (1983), 179-188), gpt oder/und neo enthalten. Um zu gewährleisten, daß zwischen Markerfunktion und Provirusfunktion keine Konkurrenz um die Verpackung erfolgt, sollten die Markervektoren keine Gene enthalten, die mit den Verpackungsfunktionen der Zielzellen von Stufe b interferieren.

Für Stufe b geeignete, eine Markerfunktion aber nicht mit den Verpackungsfunktionen interferierende Gene enthaltende Vektoren, vorzugsweise Plasmide, sind bekannt und im Handel erhältlich, beispielsweise pSV₂ neo (DSM 3858 P) (J. Mol.Appl.Genet 1, (1982),327-341) und pSV₂ gpt. Bevorzugt wird pSV₂ neo verwendet. Dieses Plasmid besteht im Prinzip aus einer Sequenz, die die Reproduktion in Prokaryonten regelt und einer weiteren DNA-Sequenz, welche die Expression des Markergens in Eukaryonten ermöglicht.

Geeignete Zielzellen für die Stufe b des erfindungsgemäßen Verfahrens sind bekannt. Sie enthalten die retroviralen Funktionen gag, pol und env, mindestens aber zwei dieser drei Funktionen, im Genom integriert. Bevorzugt werden Zellen, welche die Elemente von ecotropen und amphotropen Retroviren enthalten, verwendet. Ein Beispiel für eine bevorzugte ecotrope Zellinie ist die Maus-Fibroblasten-Zellinie φ2 (PSI 2/ECACC 86101501) (Cell 33(1983), 153-159). Beispiele für besonders bevorzugte amphotrope Zellen sind pA 317 (ATCC CRL 9078; Molec. and Cell. Biol. 6, No. 8, (1986) 2895-2902) und pA 12 (Molec. and Cell. Biol. 5 (1985) 431-437).

Mit aus diesen Zellen sezernierten defekten Retroviren können in Schritt c Zellen infiziert werden, die auf ihrer Oberfläche entsprechende Rezeptoren tragen. Besonders bevorzugt werden amphotrope Zellen verwendet, da mit den hieraus sezernierten Retroviren eine Vielzahl von Säugerzellen, insbesondere Primatenzellen infiziert werden können.

Nach Einführung des Provirus und des Markervektors in die Zielzelle, welche im Genom die retroviralen Replikations-und/oder Verpackungsfunktionen enthält, werden in der Zelle sowohl neue virale RNA, die das Fremdgen mitumfaßt, als auch die Strukturproteine, welche die RNA umhüllen, gebildet und auf diese Weise komplette neue Retroviren erzeugt, welche sich von dem ursprünglich eingesetzten Retrovirus durch den zusätzlichen Gehalt an dem gewünschten Fremdgen unterscheiden. Zellen, welche diese Eigenschaften besitzen, werden in Stufe c des erfindungsgemäßen Verfahrens durch Klonieren und Selektionieren nach dem Vorhandensein der Markerfunktion und, soweit die Markerfunktion gefunden ist, nach Bildung des gewünschten Stoffwechselproduktes selektioniert. Um die Selektion zu erleichtern, wird zweckmäßig ein etwa 2 bis 10facher Überschuß an Provirus gegenüber dem Markervektor für die Transfektion eingesetzt. Hierdurch wird sichergestellt, daß in der Mehrzahl der die Markerfunktion enthaltenden Zellen auch das Provirus enthalten ist. Besonders bevorzugt wird ein 4 bis 6facher Überschuß an Provirus verwendet.

Die Selektion in Stufe c erfolgt je nach Art des verwendeten Markergens. Handelt es sich beispielsweise um ein Antibiotikaresistenzgen, so erfolgt die Selektion in der dem Fachmann vertrauten Weise durch Zugabe des entsprechenden Antibiotikums zum Medium, so daß nur noch solche Zellen wachsen können, welche das Markerplasmid enthalten.

Die in Stufe c selektionierten Zellen werden gezüchtet, wobei sich im Wachstumsmedium die neugebildeten Retroviren befinden, die dann zur Infektion der eigentlichen eukaryontischen Zielzelle in Stufe d verwendet werden. Geeignete eukaryontische Zielzellen sind dabei solche, welche auf ihrer Oberfläche Rezeptoren für den verwendeten Retrovirus besitzen. Derartige Zellen sind alle diejenigen, welche von dem jeweils dem Provirus zugrundeliegenden Retrovirus infiziert werden können. Beispielsweise können Retroviren, welche aus φ 2-Zellen sezerniert werden, MLV-Rezeptor-tragende Maus-Zellen, beispielsweise EC-Cells (Embrional Carcinoma Cells (PNAS 81, (1984), 7137-7140)), Rat pituitary cells (GH4C₁, PNAS 81 (1984) 5340-5344), Mouse hepatoma cells (hepa 1-a, PNAS 83, (1986) 409-413) oder 3T3-Zellen (Cell. Vol 37, 1053-1062 (1984)),infizieren. Eine bevorzugte Maus-Zelle bei Verwendung des MoMLV-Retrovirus für das Verfahren der Erfindung ist die Zellinie NIH/3T3 (ATCC CRL 1658). Viruspartikel aus amphotropen packaging-Linien, beispielsweise pA317 ATCC CRL 9078, die ein entsprechend weiteres Wirkungsspektrum aufweisen können, z.B. sowohl humane als auch murine Zellen (Molec. and Cell Biology 5 (1985) 431-437) infizieren und zur Expression des gewünschten Stoffwechselproduktes veranlassen.

Gemäß einer bevorzugten Ausführungsform des erfindungsgemäßen Verfahrens werden in Stufe d einzelne Eukaryonten-Zellen verwendet und mit dem Retrovirus infiziert. Hierzu wird der virushaltige Überstand in Mikrotiterplatten vorgelegt und anschließend maximal eine Zelle pro Napf zugegeben. Dies läßt sich beispielsweise mittels handelsüblicher Zellsorter durchführen. Auch durch entsprechende Verdünnung einer Zellsuspension (ca. 1 Zelle/100μl) und Verteilung auf Mikrotiterplatten kann die Einzelzellablage erreicht werden. Nach ca 2 bis 3 wöchigem Wachstum und Vermehrung auf ca. 1000 bis 2000 Zellen pro Napf können die so klonierten infizierten Eukaryonten-Zellen daraufhin untersucht werden, ob sie das gewünschte Stoffwechselprodukt in besonders hohem Ausmaß produzieren. Hierzu wird üblicherweise ein Aliquot des Überstandes entnommen und mit ELISA auf das gesuchte Stoffwechselprodukt untersucht. Besonders gut produzierende Zellen (Overproducer) werden dann für die eigentliche Zellfermentation und Gewinnung des Stoffwechselproduktes, z.B. von t-PA verwendet.

Gemäß einer weiteren bevorzugten Ausführungsform der Erfindung kann der Infektionszyklus von Stufe d mehrfach wiederholt werden. Hierzu werden vorzugsweise aus den Kulturen, welche die besten Overproducer enthalten, Zellen entnommen, wieder in Näpfe von Mikrotiterplatten, die Virusüberstand enthalten, vereinzelt und kloniert. Dabei erfolgt eine Mehrfachintegration des interessierenden Fremdgens ins Zellgenom, was eine entsprechend große Bildungsrate des gewünschten Stoffwechselproduktes zur Folge hat.

Ein wesentliches Merkmal des erfindungsgemäßen Verfahrens ist darin zu sehen, daß der Stoffwechsel der Zielzelle nicht durch die Produktion von kompletten Viren oder einer Reihe der Zelle fremder Stoffwechselprodukte belastet wird. Da lediglich ein einziges Fremdgen abgeschrieben und das daraus gebildete Produkt sezerniert werden muß, erfolgt nur eine minimale Störung des eigentlichen Zellstoffwechsels, so daß die infizierten Zellinien viele Züchtungszyklen ohne genomische Veränderungen durchlaufen und gleichzeitig das gewünschte Stoffwechselprodukt in hohen Ausbeuten produzieren. Da die eukaryontische Zielzelle keine Retrovirusfunktionen im Genom enthält, kann sie auch keine Hüllproteine und reverse Transkriptase mehr bilden, so daß auch keine neuen Viren mehr entstehen. Daher wird auch eine Belastung des Zellstoffwechsels durch Bildung neuer Viren vermieden. Dies beruht darauf, daß der im Verfahren der Erfindung verwendete Provirus nicht alle Informationen zur Bildung eines neuen Virus enthält, sondern lediglich durch die im Genom der in Stufe b infizierten Zellen enthaltenen Virusfunktionen komplettiert werden kann. Der ins Genom der Wirtszelle integrierte Provirus, der gemäß der Erfindung nur aus den Anfangs-und Endsequenzen und dem interessierenden Fremdgen besteht, ergibt bei der Transkription eine Retrovirus-RNA, die zwar mit den in der Zelle - schon vorhandenen Hüllproteinen und der reverse Transkriptase nach Art eines Virus verpackt und aus der Zelle ausgeschleust wird, die jedoch nach Einführung in die Zielzelle in Stufe d, die keine Hüllproteine und reverse Transkriptase bilden kann, auch keine neuen Viren produzieren kann. Außerdem ist auch kein Markergen mehr vorhanden, welches eine zusätzliche Belastung des Zellstoffwechsels herbeiführt. Da die Zelle das gewünschte Produkt auch sezerniert, häuft es sich nicht im Zellinnern an, wodurch besonders günstige Voraussetzungen für die Weiterzüchtung der Zielzelle und Bildung des gewünschten Produktes entstehen.

Die Erfindung eignet sich generell zur Herstellung der durch Eukaryontengene codierten Stoffwechselprodukte wie z.B. Enzyme und Proteine des Blutgerinnungssystems, wie tPA, Gerinnungsfaktoren, Coenzyme, Hormone usw.

Die folgenden Beispiele erläutern die Erfindung weiter.

## Beispiel 1

A: Expression von tPA

Es wird vom Vektor pZIP neo SV (Mulligan et al.loc. cit. DSM 3859 P) ausgegangen. Dieser enthält das 5'-Element des MoMLV (Murine leukemia virus) als Hind III-Bam HI Fragment, also dem 5'-Terminus des Provirus und die LTR-Sequenzen, sowie das Xho I-Eco R I Fragment des gleichen Virus, also die Sequenzen des 3'-LTR, in einem pBR 322-Plasmid. Die Anordnung der viralen Elemente im Vektor entspricht der Orientierung im Provirus.

Zur Deletion der zwischen den beiden proviralen Elementen enthaltenen Sequenzen von pZIP neo SV wurde der Vektor mit Bam HI und Xho I gespalten. Ihre Restriktionsstellen wurden mit Klenow Polymerase behandelt und ein kb-DNA Fragment isoliert, das nach Ligation und Transformation in E.coli HB 101 (DSM 1607) einen Vektor ergab, bei dem das 5'-und das 3'-Element des Provirus direkt aneinander liegen. Dieser Vektor wird mit ZΔBX bezeichnet. Durch diese Manipulation entsteht an der Verknüpfungsstelle der beiden proviralen Elemente eine Xho I-Spaltungsstelle, die im Ausgangsvektor nicht vorhanden ist. Diese Stelle eignet sich zur Insertion zu exprimierender Fremdgene.

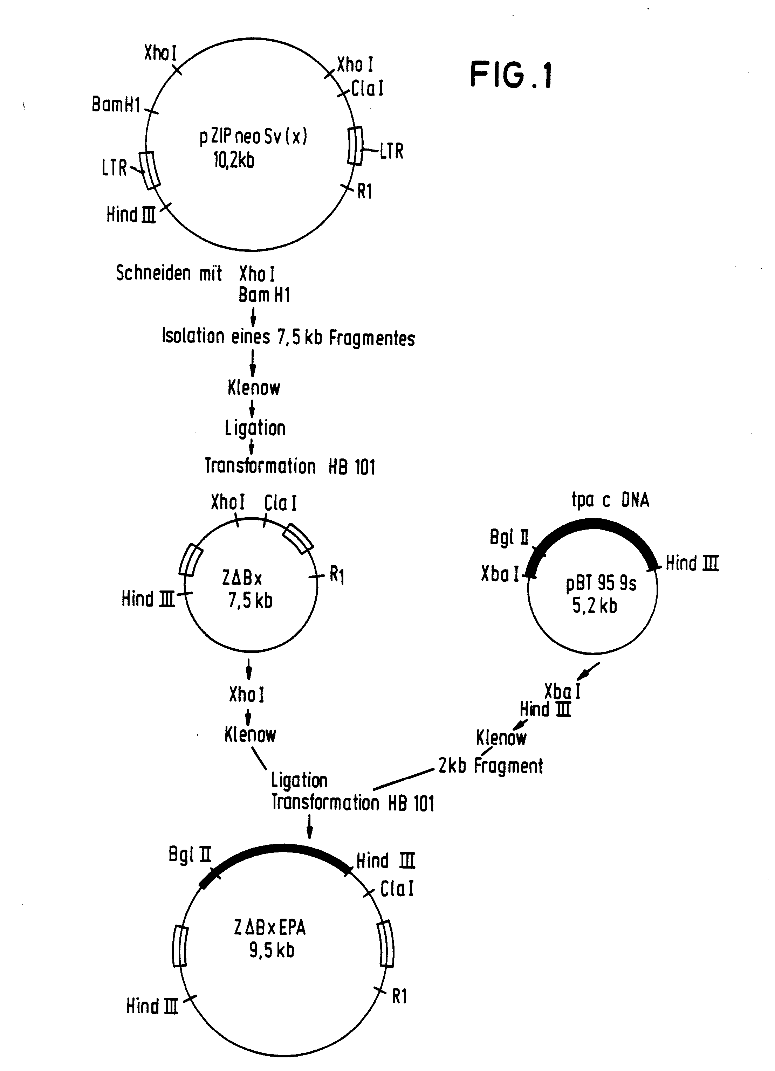

Aus dem Plasmid pBT 95 (DSM 3611P) wurde die tPA c-DNA als Xba I-Hind III Fragment isoliert. Der aus dem Vektor pZIP wie oben beschrieben erhaltene Vektor wurde mit Xho I geöffnet. Die Enden beider aufgespaltener Plasmide wurden mit Klenow Polymerase behandelt und miteinander ligiert. Man erhielt so das Vektor-Plasmid ZΔBXEPA (DSM 3860 P) (Figur 1).

B: Transfektion von φ2 Maus Fibroblasten

Eine Mischung von ZΔBXEPA-DNA und pSV2 neo (handelsübliches Markerplasmid) wurde im Verhältnis 4:1 und nach Prezipitation mit Calciumphosphat in φ2-Zellen transfiziert. Durch Selektion auf Neomycinresistenz erhält man φ2-Klone, welche das pSV2 neo Plasmid enthalten. Die so erhaltenen Klone werden auf tPA Bildung untersucht. tPA positive Klone enthalten beide Plasmide. Bei der Überprüfung der Kulturüberstände der neo$^r$-Kolonien wurde bei 20 bis 60 % der Kolonien auch tPA-Aktivität festgestellt.

Anschließend wurden die tPA-positiven Klone untersucht, ob in den Kulturüberständen Viruspartikel sezerniert werden, die die genetische Information für tPA enthalten und diese auf andere Zellen mittels retroviaraler Infektion übertragen können. Dazu wurden die Kulturüberstände durch 0,45 μ Filter gesaugt und zusammen mit 8 μg/ml Polybrene mit ca. 1000 NIH/3T3 Zellen in Kontakt gebracht. Nach 3 Stunden wurden die Überstände entfernt und die Zellen gewaschen. Dann wurden die 3T3 Zellen weiter kultiviert und nach 1 Woche die Kulturüberstände auf Gehalt an tPA mit ELISA untersucht. Dabei erwies sich ca. die Hälfte der Kulturen als positiv. Dies bedeutet, daß die Hälfte der analysierten φ2 tPA Klone in der Lage waren, Viruspartikel zu produzieren, mit deren Hilfe die genetische Information für tPA in Forma von RNA auf andere Zellen übertragen werden kann.

## Beispiel 2

Es wurde wie im Beispiel 1 beschrieben verfahren, die Kulturüberstände der auf Neomycinresistenz und tPA-Bildung selektionierten Klone wurden nach Filtration durch den 0,45 μ Filter in 200 μl Portionen in die Näpfe von 96er Mikrotiter-Platten gegeben. In jeden dieser Näpfe wurde mit Hilfe eines Fluoreszenz aktivierten Zellsorters (FACS) jeweils eine einzelne 3T3 Zelle abgelegt und dann wie in Beispiel 1 kultiviert und nach einer Woche mit ELISA auf tPA getestet. Mehr als 50 % der gebildeten Klone erwiesen sich als tPA-Bildner.

Dieses Verfahren erlaubt es, bei niedrigen Virustitern eine hohe Klonausbeute von Transduktanten zu erzielen. Außerdem wird durch dieses Einzelzellentransfektionsverfahren ein relativ hoher Virusüberschuß pro Zelle erzielt. Transduktion, Klonierung und Screening verlaufen hierbei in einem Schritt.

Die Mikrotiter-Platten wurden dann bei 37°C inkubiert und nach Expansion bis zu einer Zelldichte von $10^3$ bis $10^4$ Zellen/Napf wird das Medium gewechselt und im neuen Medium dann die Neusynthese von tPA im Überstand mit ELISA kontrolliert. Bei der großen Mehrheit der erhaltenen Klone ergab sich dabei eine tPA-Bildung.

Durch quantitative Bestimmung der in den einzelnen Näpfen gebildeten tPA werden die Klone mit den besten Produktionsraten festgestellt und dann einem weiterem Infektionszyklus in gleicher Weise, also durch Einzelzelleninfektion mittels FACS unterworfen. Hierbei wird die Kopienzahl an tPA cDNA erhöht und durch Wiederholung dieses Zyklus läßt sich die Kopienzahl sukzessiv weiter erhöhen und damit auch eine entsprechende Expressionssteigerung erzielen, ohne daß die Zellen mit irgendwelchen Drogen beeinflußt werden müssen.

## Beispiel 3

Expression von Faktor IX

Das Plasmid ZΔBX (Beispiel 1) wird mit Xho I gespalten und mit Klenow Polymerase behandelt. Durch Spaltung des Plasmids pSPATF 9 (DSM 3862 P) mit Bgl II und Ncõ I wird die cDNA von Faktor IX erhalten. Nach Behandlung mit Klenow-Enzym kann ein 2 Kb-Fragment isoliert und in den vorbereiteten Vektor ZΔBX ligiert werden.

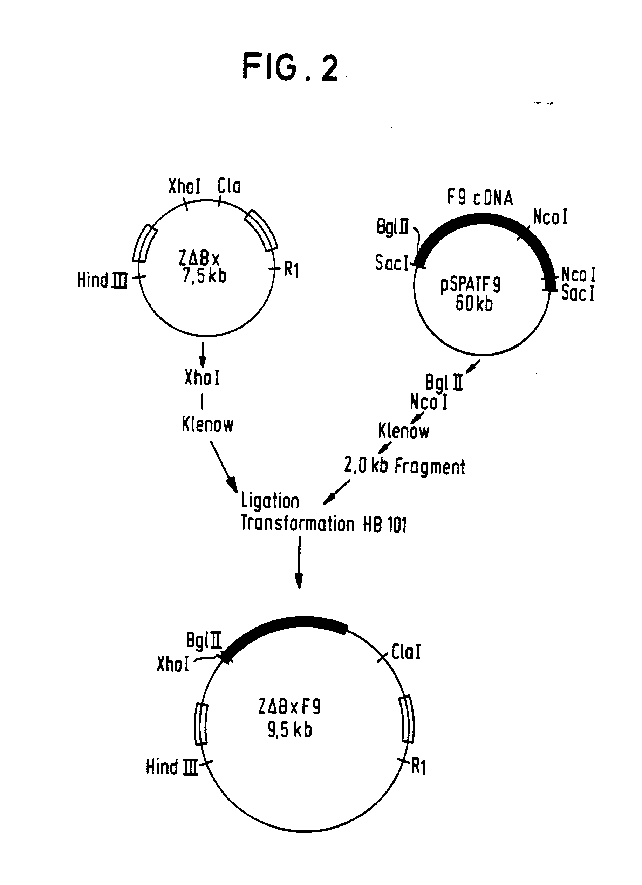

Der so erhaltene Vektor wird in E.coli HB 101 transformiert und daraus ein Plasmid gewonnen, welches die Faktor IX cDNA in der richtigen Orientierung zwischen den Anfangs-und Endsequenzen der proviralen Elemente enthält (ZΔBXF9, DSM 3861 P) (Figur 2).

Wie in Beispiel 1 beschrieben wird das so erhaltene Plasmid im Verhältnis 4:1 mit pSV2 neo Plasmid in φ2-Zellen transfektiert.

Durch Selektion mit G 418 (Neomycinderivat, Ann.Rev. Microbiol. 32 (1978) 469-518)erhält man Neomycin resistente Kolonien. Diese werden auf Sekretion auf Faktor IX-Protein mit ELISA ins Medium überprüft. Positive Klone werden wie in Beispiel 2 beschrieben im 3T3-Transduktionstest auf die Bildung infektiöser Viruspartikel analysiert und damit die Faktor IX-Virus produzierenden Zellen identifiziert. Mit den Überständen der Faktor IX-Virus produzierenden Zellen werden Maus-Fibroblasten transduziert, die in Abhängigkeit von Vitamin K aktives Faktor IX-Protein ins Kulturmedium sezernieren.

## Ansprüche

1. Verfahren zur gentechnologischen Herstellung von Stoffwechselprodukten in Eukaryontenzellen durch Einführung eines das gewünschte Stoffwechselprodukt kodierenden Fremdgens mit Hilfe eines retroviralen Provirus als Vektor in eine Eukaryontenzelle und Expression des durch das Fremdgen kodierten Produktes,
**dadurch.gekennzeichnet,**
daß man

a) das zu exprimierende Fremdgen in einen in einem Vektor integrierten Provirus einbaut, der keine funktionsfähigen Gene für die Replikations-und Verpackungsfunktionen gag, pol oder und env mehr enthält,

b) den so erhaltenen, den Provirus enthaltenden Vektor und einen weiteren, mindestens eine Markerfunktion, aber keine mit den Verpackungsfunktionen interferierenden Gene enthaltenden Vektor in eine eukaryontische Zelle einführt, die im Genom die retroviralen Gene der Replikations-und Verpackungsfunktionen gag, pol oder und env enthält, wobei Provirus und retrovirale Gene im Genom zusammen einen kompletten Retrovirus kodieren müssen,

c) die transfektierte Zelle kloniert und nach Markerfunktion und gewünschtem Stoffwechselprodukt selektioniert,

d) mit den im Wachstumsmedium der selektionierten Zellen gebildeten Retroviren eine Eukaryontenzelle infiziert und das gewünschte Stoffwechselprodukt aus der infizierten Zelle oder ihrem Züchtungsmedium gewinnt.

2. Verfahren nach Anspruch 1,
**dadurch gekennzeichnet,**
daß man einen Provirus verwendet, der in einem Prokaryontenplasmid eingebaut vorliegt.

3. Verfahren nach Anspruch 1 oder 2,
**dadurch gekennzeichnet,**
daß man in Stufe b einen 2-bis 10-fachen Überschuß an Provirus enthaltendem Vektor gegenüber dem Markervektor einsetzt.

4. Verfahren nach Anspruch 3,
**dadurch gekennzeichnet,**
daß man als Zelle eine ecotrope packaging Linie verwendet.

5. Verfahren nach Anspruch 4,
**dadurch gekennzeichnet,**
daß man in Stufe b als Zelle PSI 2 (ECACC 86101501) verwendet.

6. Verfahren nach Anspruch 5,
**dadurch gekennzeichnet,**
daß man als Zelle eine amphotrope packaging Linie verwendet.

7. Verfahren nach Anspruch 6,
**dadurch gekennzeichnet,**
daß man als Zelle pA 317 (ATCC CRL 9078) verwendet.

8. Verfahren nach Anspruch 1 bis 7,
**dadurch gekennzeichnet,**
daß man in Stufe d als Eukaryonten-Zelle eine 3T3-Zelle (ATCC CRL 1658) verwendet.

9. Verfahren nach Anspruch 1 bis 8,
**dadurch gekennzeichnet,**
daß man als retroviralen Provirus MMTV, SSFV, SNV, MoMLV oder MSV verwendet.

10. Verfahren nach Anspruch 1 bis 9,
**dadurch gekennzeichnet,**
daß man in Stufe b als keine mit den Verpackungsfunktionen interferierenden Gene enthaltenden Vektor ein eukaryontisches Plasmid verwendet.

11. Verfahren nach Anspruch 10,
**dadurch gekennzeichnet,**
daß man das Plasmid $SV_2$ neo (DSM 3858 P) verwendet.

FIG.1

# FIG.2